# EUROPÄISCHE PATENTSCHRIFT

(11) **EP 2 203 732 B1**
(45) Veröffentlichungstag und Bekanntmachung des Hinweises auf die Patenterteilung: **30.03.2011**
(21) Anmeldenummer: 08801260.4
(22) Anmeldetag: 03.09.2008
(51) Int. Cl.: G01N 1/12

(54) **SONDE ZUR ENTNAHME VON SCHLACKENPROBEN**
PROBE FOR TAKING SLAG SAMPLES
SONDE POUR PRÉLEVER DES ÉCHANTILLONS DE LAITIER

(30) Priorität: 06.09.2007 DE 102007042261; 02.07.2008 DE 102008031390
(43) Veröffentlichungstag der Anmeldung: 07.07.2010
(73) Patentinhaber: Georgsmarienhütte GmbH, 49124 Georgsmarienhütte (DE); Elkmene Hellenic Centre For Research S.A., 177 78 Athens (GR); University Of Patras, 265 04 Rion, Patras (GR); Sidenor S.A., 152 31 Halandri (GR)
(72) Erfinder: PAPAMANTELLOS, Dimitrios, C., GR-115 21 Athen (GR); SKARMOUTSOS, Dionysios, GR-113 63 Athen (GR); CONTOPOULOS, Ioannis, GR-185 39 Piraeus (GR); PALAGAS, Christos, GR-320 00 Thebes (GR); BOUGANOSOPOULOS, Giorgios, GR-412 22 Larissa (GR); KOLM, Ingo, 49124 Georgsmarienhütte (DE)
(74) Vertreter: Röther, Peter
(86) Internationale Anmeldenummer: PCT/DE2008/001453
(87) Internationale Veröffentlichungsnummer: WO 2009/030206

(56) Entgegenhaltungen:
- EP-A- 1 329 704
- WO-A-00/73765
- DE-A1- 1 773 163
- DE-A1- 2 946 429
- DE-A1- 3 901 673
- DE-U- 7 329 884
- GB-A- 1 572 122
- SU-A1- 1 252 041

## Beschreibung

Die Erfindung betrifft eine Sonde zur Entnahme von Schlackenproben aus flüssigen Schlacken der Metall-(insbesondere Stahl-)Erzeugung mit einer flachzylindrigen Probenkavität, die über einen Kanal mit einem Einlauftrichter verbunden ist, wobei die Kavität zumindest umfangseitig durch einen Metallring begrenzt ist und zumindest eine der Ober- und Unterseiten der Kavität durch ein metallisches Abkühlelement gebildet ist.

Eine derartige Sonde ist beispielsweise aus der DE 197 52 743 C5 bekannt, die aus einem nach unten in die flüssige Schlacke eintauchenden Einlaufzylinder besteht, durch den die Schlacke über einen Einlauf in die Probenkavität eindringt. Der obere Kavitätsabschluss ist dabei durch eine dünnwandige Abkühlplatte gebildet.

Gehalten wird der Trichter und die Kavität in einer Pappröhre.

Die Kavität ist umfangseitig durch einen Ring begrenzt.

Bei der vorbekannten Sonde ist es zum einen nachteilig, dass nach der Probenahme keines der Sondenteile wieder verwendet werden kann. Darüber hinaus ist durch die dünnwandige Abkühlplatte ein schnelles Abkühlen der Probe nach der Entnahme nicht gewährleistet, so dass beim Erstarrungsvorgang in der Probe Inhomogenitäten auftreten können, die bei einer anschließenden Analyse, insbesondere durch die so genannte Laser- induzierte Spektralanalyse (LIBS), keine aussagekräftigen Ergebnisse zulassen.

Zur Optimierung der metallurgischen Reaktionen ist es von grosser Bedeutung, dass während des Prozesses neben der in kurzer Zeit zur Verfügung stehenden Metallanalyse auch eine Schlackenanalyse zeitnah vorhanden ist. Die heutige Praxis der Schlackenanalyse mittels Röntgenfluoreszenzspektroskopie (XRF) ist aufgrund des hohen Zeitaufwands für Probenvorbereitung und Analyse dafür nicht geeignet.

Als Alternative zur erwähnten Röntgenfluoreszenzspektroskopie (XRF) kommt die oben genannte LIBS-Methode in Frage, wobei diese Methode jedoch Proben mit einer möglichst homogenen Erstarrungsstruktur und Oberflächenbeschaffenheit erfordert.

Mit der vorbekannten Sonde können diese Voraussetzungen erfahrungsgemäss nicht sicher erreicht werden.

Der Erfindung liegt daher die Aufgabe zugrunde, eine Sonde der eingangs genannten Art anzugeben, die zum größten Teil mehrfach wieder verwendet werden kann und die eine Abkühlgeschwindigkeit erlaubt, die eine schnelle Verfügbarkeit einer Schlackenprobe für die Analyse gewährleistet sowie eine für die LIBS- Methode erforderliche Homogenität und Oberflächenbeschaffenheit der Probe sicherstellt.

Die Erfindung löst diese Aufgabe gemäß dem kennzeichnenden Teil des Anspruchs 1 dadurch, dass der Einlauftrichter und der Kanal jeweils in ein Metallsegment eingeformt sind und das Kanalsegment auf dem Metallring aufsitzt, der wiederum auf dem Abkühlelement aufsitzt, welches aus einem Kupfersegment gebildet ist, dessen Dicke ein mehrfaches der Kavitätshöhe aufweist, und diese Elemente und Segmente in umgekehrter oben angegebener Reihenfolge in ein einseitig offenes metallisches Gehäuse einschiebbar und aus diesem heraus bewegbar sind, wozu eine Einrichtung zum Ausstoßen der im Gehäuse befindlichen Elemente und Segmente vorgesehen ist.

Das metallische Gehäuse besteht vorzugsweise aus Stahl.

Die in dieses Gehäuse eingeschobenen einzelnen Segmente bilden die Sonde mit Einlauftrichter, Einlaufkanal, Kavität und Abkühlplatte, wobei der Begriff Abkühlplatte im vorliegenden Fall bedeutet, dass hier im Gegensatz zum Stand der Technik ein dickwandiges Element vorgesehen ist, das die Probenwärme schneller abführen kann. Die Dimensionierung und Materialwahl des Kühlelements wurde durch wärmetechnische Berechnungen abgesichert .

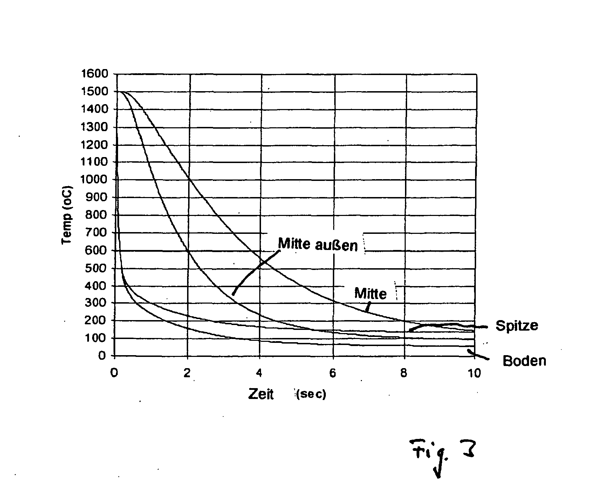

Rasche Abkühlung der Schlackeprobe auf eine Temperatur unterhalb von 500°C ist von erheblicher Wichtigkeit für ihre Homogenität und Stabilität. Verschiedene alternative Sondenausgestaltungen sind mit Hilfe der "Commercial computational fluid dynamics (CFD)" Software Paket ANSYS/Fluent + Gambit entwickelt worden. Wir entdeckten den Einfluss verschiedener Materialkomponenten (Stahl, Kupfer) sowie Komponentenabmessungen für die Abkühlrate und wählten die Kombination, die eine schnellere und homogenere Abkühlung entlang dem größt möglichen Teil der Schlackeprobe zusammen mit der mechanischen Stabilität der Sonde in der rauen Umgebung eines Stahlofens garantierte. Typische Abkühlkurven verschiedener Teile der Sonde sind in dem als Figur 3 beigefügten Diagramm für die endgültige Sondenausgestaltung dargestellt.

Nach dem Eintauchen der erfindungsgemäßen Sonde in die flüssige Schlacke füllt sich die Kavität über den Einlauftrichter und den Kanal.

Nach dem Herausziehen der Sonde aus der flüssigen Schlacke kühlt die genommene Probe in der Kavität schnell ab. Durch die Einrichtung zum Ausstoßen der im Gehäuse befindlichen Elemente und Segmente wird die genommene und erstarrte Probe zugänglich, die sofort für die Analyse zur Verfügung steht.

Alle Elemente und Segmente bis auf den die Probe umgebenden Metallring , der auch als Armierung für den Probenkörper dient und somit ein verlorenes Formelement darstellt, können für die nächste Probenahme wieder verwendet werden.

Aufgrund der oben erwähnten wärmetechnischen Berechnungen wurde auch das Kanalsegment aus Kupfer gefertigt, um eine noch raschere und homogenere Abkühlung der Probe zu gewährleisten.

Auf diese Weise ist die Kavität von allen Seiten durch ein Material gebildet, das eine sehr gute Wärmeleitung aufweist und dadurch den Abkühlvorgang unterstützt.

Die glatte Analysenfläche der Probe wird durch die Oberfläche des aus Kupfer bestehenden Abkühlelements gebildet.

Um der Probe bei der Entformung und nach der Entformung eine noch bessere Stabilität zu verleihen, ist gemäß Anspruch 3 vorgesehen, dass der Metallring mit einem Deckel aus gleichem Material versehen ist, in dem eine Öffnung vorhanden ist, deren Querschnitt mit dem Kanalquerschnitt übereinstimmt und welche mit dem Kanal fluchtet.

Gemäß Anspruch 4 besteht die Einrichtung zum Ausstoßen aus einem durch den Boden des Gehäuses nach außen vorstehenden Konus aus Stahl, der in einer ebenfalls konusförmigen Öffnung im Boden gehalten ist, wobei die ins Behälterinnere weisende Konusbasis mit der auf dem Behälterboden stehenden Seite des Abkühlelements zusammenwirkt.

Nach der Probenahme und dem anschließenden Abkühlvorgang wird der Konus in das Gehäuse getrieben, wodurch die einzelnen Elemente und Segmente aus dem Gehäuse heraus gedrückt werden.

Nach erfolgter Probenentnahme wird zunächst der Konus wieder in den Boden des Gehäuses eingesetzt, danach folgen die restlichen Elemente und Segmente, wobei lediglich ein neuer Probenring eingesetzt werden muss.

Zum Schutz des Abkühlelementes und zur gleichmässigen Verteilung der Kraft beim Eintreiben des Konus in das Gehäuse ist gemäß Anspruch 5 zwischen Abkühlelement und Konusbasis eine Stahlscheibe im Gehäuse vorgesehen.

In einer bevorzugten Ausführungsform ist gemäß Anspruch 6 vorgesehen, dass das Gehäuse seitlich von einem Stahlmantel umgeben ist, dessen Innenseite mit der Außenseite des Behälters einen in sich geschlossenen Ringraum bildet, in den auf einer Seite eine Leitung für die Zufuhr eines Kühlmediums mündet und aus dem an anderer Stelle eine Leitung für die Ableitung des Kühlmediums abgeht.

Durch Umspülung des Gehäuses mit einem Kühlmedium, insbesondere Gas, bereits während der Probenahme wird der Abkühlvorgang der Probe weiter beschleunigt.

Gemäß Anspruch 7 ist vorgesehen, dass die Leitungen - entsprechend den örtlichen Gegebenheiten bei der Probenahme am jeweiligen metallurgischen Aggregat - in einem geeigneten Winkel zur Behälterachse verlaufen und eine der Leitungen als Handhabe für die Sonde dient. Die Leitungen bestehen aus stabilen Rohren und weisen somit eine ausreichende Stabilität bei der Handhabung der Sonde auf.

Gemäß Anspruch 8 ist vorgesehen, dass der Einlauftrichter oberhalb der Kavität angeordnet ist. Hierdurch ist gewährleistet, dass beim Eintauchen der Sonde in die flüssige Schlacke der Mitarbeiter von oben den Einfüllvorgang genau beobachten kann, so dass ein zu tiefes Eintauchen der Sonde - eventuell bis in die Metallschmelze selbst- ausgeschlossen ist. Gemäß Anspruch 9 ist vorgesehen, dass die Sonde durch eine rotationssymmetrische Ausgestaltung gekennzeichnet ist, wobei die Gehäuselängsachse die Symmetrieachse darstellt. Die Herstellung und Handhabung einer derartigen Sonde ist somit besonders einfach und unaufwendig.

Die Erfindung wird im folgenden anhand von Zeichnungen dargestellt und erläutert.

Es zeigen:
- Fig. 1: Sonde im Schnitt mit Gaskühlung
- Fig. 2: innerer Aufbau der Sonde in Explosionsdarstellung

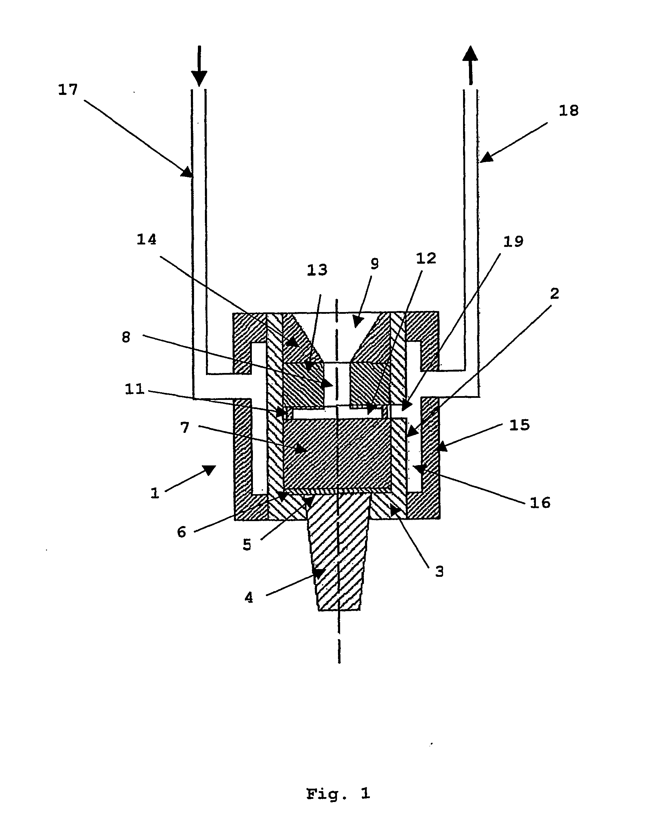

In der Figur 1 ist eine Sonde zur Entnahme einer Schlackenprobe aus flüssigen Schlacken der Metall- (insbesondere Stahl-) Erzeugung dargestellt und allgemein mit dem Bezugszeichen 1 versehen.

Sie besteht in der dargestellten Ausführungsform aus einem Gehäuse 2, das vorzugsweise zylindrisch ausgebildet ist und über einen Boden 3 verfügt, in dem eine konisch nach außen zulaufende Öffnung vorgesehen ist, in die ein Stahlkonus 4 eingesetzt ist, dessen Basisfläche 5 mit der inneren Bodenfläche bündig abschließt.

Auf der Bodenfläche ist eine Stahlscheibe 6 angeordnet, auf der ein Kupferzylinder 7 angeordnet ist. Auf die obere Stirnfläche des Kupferzylinders 7 ist ein Metallring 11 (aus Stahl oder Kupfer)aufgesetzt, auf dem ein Ring 13 aus Kupfer sitzt, der einen Kanal 8 umgibt, der die Verbindung zwischen einem Einlauftrichter 9 und einer durch den Ring 11 gebildete Probenkavität 12 bildet.

Der Einlauftrichter 9 ist in ein Segment 14 beispielsweise aus Stahl eingeformt, welches auf dem Kupferring 13 mit dem Kanal 8 aufsitzt. Einlaufsegment 14 und Kupferring 13 sind vorzugsweise zweiteilig ausgeführt, um eine bessere Trennung der in der Kavität 12 befindlichen Schlackenprobe von den darüber angeordneten Teilen ohne Beschädigung der Schlackenprobe zu ermöglichen.

Dass der die Schlacke sammelnde und kühlende Hohlkonus an der Spitze der Sonde aus zwei Teilen (Stahl oben und Kupfer unten) besteht, hat einen Grund. Das Problem mit der ursprünglichen einstückigen Ausgestaltung war, dass, wenn die gesammelte Schlacke erstarrte, es keinen leichten Weg gab, den obersten Teil von der Probe zu trennen, ohne die glasartige Probe schwer zu beschädigen. Die zweiteilige Ausgestaltung garantiert, dass sobald die Probe aus dem Hauptkörper der Sonde herausgezogen ist und abkühlt, die Kontraktion des aus Stahl bestehenden oberen Teilkeils einen kleinen Bruch in der Schlacke erzeugt, der es erlaubt, dass die Schlacke an dieser Position leicht bricht und im folgenden der aus Kupfer bestehende untere Teil leicht herausgezogen werden kann.

Umgeben ist das Gehäuse 2 von einem Stahlmantel 15, dessen Innenwand mit der Außenwand des Gehäuses 2 einen in sich geschlossenen Ringraum 16 bildet. In diesen Ringraum 16 mündet einerseits eine Zufuhrleitung 17 für ein Kühlmedium, insbesondere Gas. Auf der anderen Seite führt eine Abführleitung 18 aus dem Ringraum 16 das Kühlmedium heraus. Die Leitungen 17 und 18 bestehen aus Metallrohren, wobei die Zuführleitung 17 gleichzeitig als Handhabe für die Sonde dient.

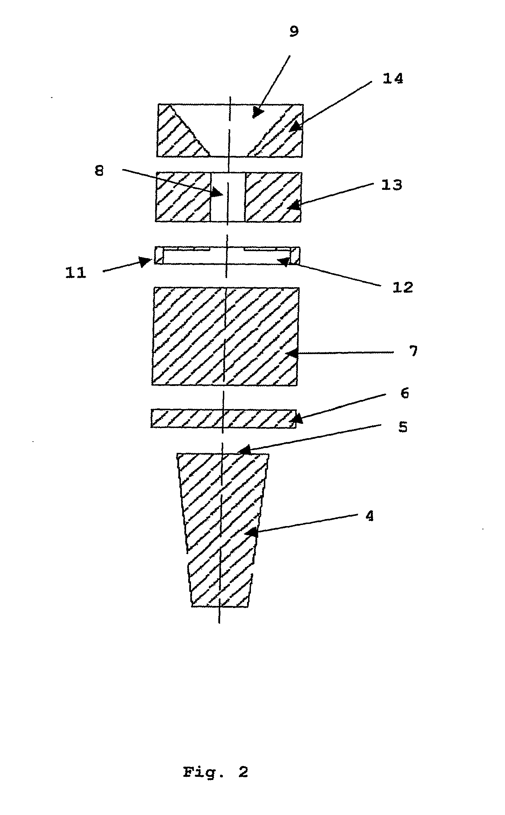

Der innere Aufbau im Gehäuse 2 ist noch einmal im Detail in Figur 2 dargestellt. Hierbei sind die einzelnen Segmente und Elemente, die die Kavität 12 bilden bzw. umschließen wie in Figur 1 bezeichnet.

Die auseinander gezogene Darstellung der einzelnen Teile verdeutlicht, dass dieselben eine zerlegbare und wieder montierbare und somit auch wieder verwendbare Sonde ergeben.

Lediglich der Metallring 11 mit seinem oberen Abschlussdeckel muss für jede Probenahme ersetzt werden, da diese Elemente nach der Entformung der Probe als Stabilisierung derselben dienen.

In einer einfacheren Ausführungsform ohne Gaskühlung wird der Stahlmantel 15 mit den Leitungen 17 und 18 weggelassen.

Eine nicht dargestellte Handhabe wird in diesem Falle bei 19 am Gehäuse 2 befestigt. Die Öffnung 19 dient gleichzeitig als Entlüftung der Kavität während der Befüllung mit der flüssigen Schlacke. Durch entsprechende Toleranzen zwischen dem Metallring 11 und dem Gehäuse 2 ist eine für die Entlüftung ausreichende Verbindung der Kavität mit der Öffnung 19 gewährleistet.

## Patentansprüche

1. Sonde zur Entnahme von Schlackenproben aus flüssigen Schlacken der Metall- (insbesondere Stahl-) Erzeugung mit einer flachzylindrigen Probenkavität, die über einen Kanal mit einem Einlauftrichter verbunden ist, wobei die Kavität zumindest umfangseitig durch einen Metallring begrenzt ist und zumindest eine der Ober- und Unterseiten der Kavität durch ein metallisches Abkühlelement gebildet ist, **dadurch gekennzeichnet,**
**dass** der Einlauftrichter (9) und der Kanal (8) jeweils in ein Metallsegment (13, 14) eingeformt sind und das Kanalsegment (13) auf dem Metallring (11) aufsitzt, der wiederum auf dem Abkühlsegment (7) aufsitzt, welches aus einem Kupferelement gebildet ist, dessen Dicke ein mehrfaches der Kavitätshöhe aufweist und diese Elemente und Segmente (7, 11, 13, 14) in umgekehrter oben angegebener Reihenfolge in ein einseitig offenes metallisches Gehäuse (2) einschiebbar und aus diesem heraus bewegbar sind, wozu eine Einrichtung (4) zum Ausstoßen der im Gehäuse befindlichen Elemente und Segmente (7, 11, 13, 14) vorgesehen ist.

2. Sonde nach Anspruch 1,
**dadurch gekennzeichnet,**
**dass** das Einlauftrichtersegment (14) aus Stahl oder Kupfer, das Kanalsegment (13) aus Kupfer und der Metallring (11) aus Stahl oder Kupfer gebildet ist.

3. Sonde nach Anspruch 1 oder 2,
**dadurch gekennzeichnet,**
**dass** der Metallring (11) mit einem Deckel aus gleichem Material versehen ist, in dem eine Öffnung vorhanden ist, deren Querschnitt mit dem Kanalquerschnitt übereinstimmt und welche mit dem Kanal (8) fluchtet.

4. Sonde nach einem der Ansprüche 1 bis 3,
**dadurch gekennzeichnet,**
**dass** die Einrichtung zum Ausstoßen aus einem durch den Boden des Gehäuses nach außen vorstehenden Konus (4) aus Stahl besteht und in einer ebenfalls konusförmigen Öffnung im Boden gehalten ist, wobei die ins Behälterinnere weisende Konusbasis (5) mit der auf dem Behälterboden stehenden Seite des Abkühlelements (7) zusammenwirkt.

5. Sonde nach Anspruch 4,
**dadurch gekennzeichnet,**
**dass** zwischen Abkühlelement (7) und Konusbasis (5) eine Stahlscheibe (6) vorgesehen ist.

6. Sonde nach einem der Ansprüche 1 bis 5,
**dadurch gekennzeichnet,**
**dass** das Gehäuse (2) seitlich von einem Stahlmantel (15) umgeben ist, dessen Innenseite mit der Außenseite des Behälters einen in sich geschlossenen Ringraum (16) bildet, in den auf einer Seite eine Leitung (17) für die Zufuhr eines Kühlmediums mündet und aus dem an anderer Stelle eine Leitung (18) für die Ableitung des Kühlmediums abgeht.

7. Sonde nach Anspruch 6,
**dadurch gekennzeichnet,**
**dass** die Leitungen (17,18) - entsprechend den örtlichen Gegebenheiten bei der Probenahme am jeweiligen metallurgischen Aggregat - in einem geeigneten Winkel zur Behälterachse verlaufen und eine der Leitungen als Handhabe für die Sonde (1) dient.

8. Sonde nach einem der Ansprüche 1 bis 7,
**dadurch gekennzeichnet,**
**dass** der Einlauftrichter (9) oberhalb der Kavität (12) angeordnet ist.

9. Sonde nach einem der Ansprüche 1 bis 8,
**gekennzeichnet durch** eine rotationssymmetrische Ausgestaltung, wobei die Gehäuselängsachse die Symmetrieachse darstellt.

## Claims

1. A probe for taking slag samples from liquid slag during metal (in particular steel) production, said probe comprising a flat cylindrical sample cavity connected via a channel to a feeding funnel, wherein the cavity is circumferentially delimited by a metal ring, and at least one of the upper and lower sides of the cavity is formed by a metallic cooling element, **characterized in that** the feeding funnel (9) and the channel (8) each are integrally formed into a metal segment (13, 14) and the channel segment (13) rests on the metal ring (11) which, in turn, rests on the cooling segment (7) formed from a copper element having a thickness corresponding to a multiple of the height of the cavity, and said elements and segments (7, 11, 13, 14) can be inserted in the reversed above-mentioned sequence into a metallic housing (2) open on one side and can be moved out of the same, wherefore a device (4) is provided for ejecting the elements and segments (7, 11, 13, 14) located in the housing.

2. The probe according to claim 1,
**characterized in**
**that** the feeding funnel segment (14) is formed from steel or copper, the channel segment (13) is formed from copper, and the metal ring (11) is formed from steel or copper.

3. The probe according to claim 1 or claim 2,
**characterized in that** the metal ring (11) is provided with a cover which is made from the same material and in which an opening is present, the cross-section of which corresponds to the channel cross-section and which is aligned with the channel (8).

4. The probe according to the claims 1 to 3,
**characterized in that** the device for ejecting consists of a cone (4) made of steel projecting outwardly through the bottom of the housing and is retained in an opening in the bottom, which opening is also cone-shaped, wherein the cone base (5) pointing into the container interior interacts with the cooling element's (7) side standing on the container bottom.

5. The probe according to claim 4,
**characterized in**
**that** between cooling element (7) and cone base (5), a steel disk (6) is provided.

6. The probe according to any one of the claims 1 to 5,
**characterized in**
**that** the housing (2) is laterally enclosed by a steel casing (15), the inner side of which forms a closed annular space (16) with the outer side of the container, into which annular space, a line (17) opens out on one side for feeding a cooling medium, and from which a line (18) extends from another position for discharging the cooling medium.

7. The probe according to claim 6,
**characterized in**
**that** the lines (17, 18) - according to the local conditions during the sampling at the respective metallurgical aggregate - run at a suitable angle to the container axis, and one of the lines serves as handle for the probe (1).

8. The probe according to any one of the claims 1 to 7,
**characterized in**
**that** the feeding funnel (9) is arranged above the cavity (12).

9. The probe according to any one of the claims 1 to 8,
**characterized by** a rotationally symmetric configuration, wherein the longitudinal housing axis represents the axis of symmetry.

## Revendications

1. Sonde pour prélever des échantillons de laitier à partir de laitiers liquides pendant la production de métal (en particulier d'acier), comportant une cavité d'échantillon cylindrique plate qui est reliée à une trémie d'alimentation par l'intermédiaire d'un canal, dans laquelle la cavité est délimitée, au moins sur sa circonférence, par une bague de métal et au moins un des côtés supérieur et inférieur est formé par un élément de refroidissement métallique, **caractérisée en ce que** la trémie d'alimentation (9) et le canal (8) sont formés chacun dans un segment métallique (13, 14) et le segment de canal (13) est en appui sur la bague de métal (11), laquelle est à son tour en appui sur le segment de refroidissement (7) qui est formé d'un élément en cuivre dont l'épaisseur est un multiple de la hauteur de cavité, et ces éléments et segments (7, 11, 13, 14) peuvent être introduits dans l'ordre inverse de celui indiqué ci-dessus dans un châssis métallique ouvert d'un côté (2) et peuvent en être retirés, des moyens (4) étant prévus pour éjecter les éléments et segments (7, 11, 13, 14) se trouvant dans le châssis.

2. Sonde selon la revendication 1, **caractérisée en ce que** le segment de trémie d'alimentation (14) est constitué d'acier ou de cuivre, le segment de canal (13) de cuivre et la bague de métal (11) d'acier ou de cuivre.

3. Sonde selon la revendication 1 ou 2, **caractérisée en ce que** la bague de métal (11) est munie d'un couvercle fait du même matériau, ledit couvercle comportant une ouverture dont la section transversale correspond à la section transversale de canal et qui est en alignement avec le canal (8).

4. Sonde selon l'une quelconque des revendications 1 à 3, **caractérisée en ce que** les moyens d'éjection sont constitués d'un cône (4) en acier faisant saillie vers l'extérieur à travers le corps du châssis, et sont maintenus dans le corps dans une ouverture également conique, la base de cône (5) dirigée vers l'intérieur de conteneur coopérant avec le côté de l'élément de refroidissement (7) situé sur le corps de récipient.

5. Sonde selon la revendication 4, **caractérisée en ce qu'**un disque d'acier (6) est prévu entre l'élément de refroidissement (7) et la base de cône (5).

6. Sonde selon l'une quelconque des revendications 1 à 5, **caractérisée en ce que** le châssis (2) est latéralement entouré d'une enveloppe en acier (15) dont le côté intérieur forme un espace annulaire fermé (16) avec le côté extérieur du récipient, espace dans un côté duquel débouche une conduite (17) pour l'alimentation d'un fluide de refroidissement et duquel ressort, à un autre endroit, une conduite (18) pour l'évacuation du fluide de refroidissement.

7. Sonde selon la revendication 6, **caractérisée en ce que** les conduites (17, 18) - en fonction des données locales lors du prélèvement d'échantillon dans l'agrégat métallurgique respectif - s'étendent à un angle approprié par rapport à l'axe d'un récipient et l'une des conduites sert de prise pour la sonde (1).

8. Sonde selon l'une quelconque des revendications 1 à 7, **caractérisée en ce que** la trémie d'alimentation (9) est agencée au-dessus de la cavité (12).

9. Sonde selon l'une quelconque des revendications 1 à 8, **caractérisée par** un agencement à symétrie de rotation, l'axe longitudinal de châssis constituant l'axe de symétrie.
